(19) Europäisches Patentamt

European Patent Office

Office européen des brevets

(11) EP 0 802 259 B1

(12) EUROPÄISCHE PATENTSCHRIFT

(45) Veröffentlichungstag und Bekanntmachung des Hinweises auf die Patenterteilung:
31.03.1999 Patentblatt 1999/13

(51) Int Cl.⁶: **C12N 15/54**, C12N 9/10, C12P 19/18

(21) Anmeldenummer: 97106357.3

(22) Anmeldetag: 17.04.1997

(54) **Cyclodextringlycosyltransferasen zur Produktion von gamma-Cyclodextrin**

Cyclodextrin glucosyltransferases for the production of gamma-cyclodextrin

Cyclodextrine glucosyltransférases pour la production de gamma cyclodextrine

(84) Benannte Vertragsstaaten:
BE DE DK FI FR GB IT NL

(30) Priorität: 18.04.1996 DE 19615336

(43) Veröffentlichungstag der Anmeldung:
22.10.1997 Patentblatt 1997/43

(73) Patentinhaber: Consortium für Elektrochemische Industrie GmbH
81379 München (DE)

(72) Erfinder:
- Schulz, Georg E., Dr.
79211 Denzlingen (DE)
- Parsiegla, Goetz, Dr.
79102 Freiburg (DE)
- Candussio, Anton, Dr.
81825 München (DE)
- Wich, Günter, Dr.
81379 München (DE)

(74) Vertreter: Potten, Holger et al
Wacker-Chemie GmbH
Zentralabteilung Patente,
Marken und Lizenzen
Hanns-Seidel-Platz 4
81737 München (DE)

(56) Entgegenhaltungen:
EP-A- 0 481 903        EP-A- 0 614 971
EP-A- 0 630 967        WO-A-91/14770
WO-A-96/33267

- DATABASE WPI Section Ch, Week 9514 Derwent Publications Ltd., London, GB; Class B04, AN 95-100943 XP002034113 & JP 07 023 781 A (OJI CORN STARCH CO LTD) , 27.Januar 1995
- DATABASE WPI Section Ch, Week 9339 Derwent Publications Ltd., London, GB; Class D16, AN 93-308317 XP002034114 & JP 05 219 948 A (UOZUMI T) , 31.August 1993
- DATABASE WPI Section Ch, Week 9313 Derwent Publications Ltd., London, GB; Class D16, AN 93-103608 XP002034115 & JP 05 041 985 A (OJI CORN STARCH CO LTD) , 23.Februar 1993
- BIOTECHNOLOGY AND APPLIED BIOCHEMISTRY, Bd. 12, Nr. 4, August 1990, ACADEMIC PRESS, INC., NEW YORK, US, Seiten 387-396, XP002034109 J. HELLMAN ET AL.: "Effects of modifications at the C-terminus of cyclomaltodextrin glucanotransferase from Bacillus circulans var. alkalophilus on catalytic activity"
- J. BIOTECHNOLOGY, Bd. 32, Nr. 3, 28.Februar 1994, ELSEVIER, AMSTERDAM,NL, Seiten 283-288, XP002034110 K.-A. SIN ET AL.: "Replacement of an amino acid residue of cyclodextrin glucanotransferase of Bacillus ohbensis doubles the production of gamma-cyclodextrin"
- BIOCHEMISTRY, Bd. 34, Nr. 10, 14.März 1995, AM. CHEM. SOC.,WASHINGTON,DC,US, Seiten 3368-3376, XP002034111 D. PENNINGA ET AL.: "Site-directed mutations in tyrosine 195 of cyclodextrin glycosyltransferase from Bacillus circulans strain 251 affect activity and product specificity"
- J. OF FERMENTATION AND BIOENGINEERING, Bd. 74, Nr. 6, - 1992 SUITA, JAPAN, Seiten 345-351, XP002034112 N. KITAMOTO ET AL.: "Cloning and sequencing of the gene encoding cyclodextrin glucanotransferase from Bacillus sp. KC201"

EP 0 802 259 B1

**Beschreibung**

Die Erfindung betrifft Cyclodextringlycosyltransferasen (CGTasen) EC 2.4.1.19 zur Produktion von $\gamma$-Cyclodextrin, Verfahren zur Herstellung von $\gamma$-Cyclodextringlycosyltransferasen und ihre Verwendung.

Cyclodextrine werden in der Regel aus Stärke oder stärkeähnlichen Substraten hergestellt. Stärke wird dabei mit CGTasen enzymatisch In Cyclodextrin (CD) umgewandelt. Aus thermodynamischen Gründen wird die Stärke unabhängig von der zur Umsetzung verwendeten CGTase hauptsächlich in $\beta$-CD umgewandelt, wenn die Reaktion bis zum Erreichen des thermodynamischen Gleichgewichts (maximaler CD-Ertrag) durchgeführt wird. In der Initialphase, zu Beginn der Stärkekonversionsreaktion jedoch unterscheiden sich die zur Konversion verwendeten Enzyme in der Zusammensetzung des primären Produktgemisches. In Abhängigkeit von dem in dieser Initialphase durch das Enzym hauptsächlich gebildeten Produkt, $\alpha$-, $\beta$- oder $\gamma$-CD, wird unterschieden zwischen $\alpha$-, $\beta$- oder $\gamma$-CGTasen.

Solche zur industriellen CD-Produktion geeigneten und auch bereits verwendeten Enzyme wurden bisher ausschließlich bei Bakterien nachgewiesen. $\alpha$-CGTasen wurden bisher ausschließlich bei Bacillus macerans, Bacillus stearothermophilus und Klebsiella oxytoca identifiziert. $\beta$-CGTasen wurden zum Beispiel bei Bacillus circulans, Bacillus megaterium, Bacillus ohbensis, Micrococcus sp. und taxonomisch nicht exakt klassifizierten alkalophilen Bacillen wie Bacillus sp. 38-2, 17-1, 1011, oder 1-1 nachgewiesen. Natürlich vorkommende Enzyme mit einer initial hohen $\gamma$-CD-Bildungsaktivität wurden aus Bacillus subtilis 313, Bacillus sp. AI-6 und Bacillus sp. 290-3 beschrieben.

Da die bei der industriellen Herstellung von Cyclodextrinen verwendeten CGTasen bei der Umwandlung von Stärke in Cyclodextrine immer Gemische aus mehreren Cyclodextrinen liefern, wurden verschiedene Verfahren zur Gewinnung reiner Cyclodextrine ($\alpha$, $\beta$ oder $\gamma$) entwickelt. Diese sind im folgenden dargestellt:

Definierte CD's können aus den Produktgemischen, z. B. aufgrund ihrer Molekulargewichtsunterschiede, chromatographisch abgetrennt werden (beschrieben beispielsweise in US-A-4,808,232).

Bei der enzymatischen Umwandlung von Stärke in Cyclodextrine werden in der Regel Komplexbildner zugesetzt, die nur mit einem definierten CD reagieren und mit ihm z. B. einen unlöslichen Komplex bilden, der dann physikalisch aus dem Reaktionsgemisch abgetrennt werden kann. Anschließend wird der Komplex aufgelöst und das homogene CD gewonnen (beschrieben beispielsweise in EP 0291067).

Durch die Zugabe eines organischen Lösungsmittels, wie z. B. Äthanol, zum Reaktionsgemisch kann die Produktzusammensetzung bei Verwendung einer $\gamma$-CGTase in Richtung $\gamma$-CD verschoben werden (J. Ferm. Bioeng. (1990) 70 (3), S. 150-154).

Bei jedem der Verfahren werden optimalerweise solche CGTasen verwendet, die eine möglichst hohe initiale Produktbildungspräferenz für das CD besitzen, das rein hergestellt werden soll.

Die Spezifität der bisher bekannten $\alpha$- und $\beta$-CGTasen ist ausreichend für eine technische Produktion der entsprechenden Cyclodextrine. Im Gegensatz dazu besitzt keine der bekannten, natürlich vorkommenden $\gamma$-CGTasen eine Produktspezifität, die eine vergleichbare technische $\gamma$-CD-Produktion ermöglicht.

Zur Herstellung von $\gamma$-CD wurde daher in CA 115:157165 vorgeschlagen, $\alpha$- und/oder $\beta$-Cyclodextrine enzymatisch durch die Zugabe des $\gamma$-CD-spezifischen Komplexbildners Glycosylglycyrrhizin, Maltose und einer CGTase in $\gamma$-CD umzuwandeln.

Eine weitere Möglichkeit zur Herstellung von $\gamma$-CD besteht darin, durch Austausch definierter Aminosäurereste die $\gamma$-CD-Spezifität einer $\beta$-CGTase so zu erhöhen, daß das mutagenisierte Enzyme vermehrt $\gamma$-CD produziert und damit zur technischen Herstellung von $\gamma$-CD verwendet werden kann. Entsprechende Mutationen sind bekannt und z. B. in DE 43 24 650 AI (entspricht US-A-5,474,917), Biochemistry. (1994) 33 (33), S. 9929-9936, Biochemistry. (1995) 34 (10), S. 3368-3376 und J. Biotech. (1994) 32, S. 283-288 beschrieben.

Solche CGTase - Derivate, die durch Mutagenese von $\beta$-CGTasen erzeugt wurden, besitzen eine erhöhte $\gamma$-CD - Spezifität und sind damit aufgrund ihrer Produktspektren prinzipiell zur technischen Herstellung von $\gamma$-CD geeignet. Nachteilig ist jedoch, daß die spezifischen Aktivitäten der zur Mutagenese verwendeten Ausgangsenzyme durch die Einführung der jeweiligen Mutationen reduziert werden. In Abhängigkeit von den eingeführten Aminosäureresten besitzen mutierte Enzyme mit einer erhöhten $\gamma$-CD - Spezifität nur noch zwischen 25 % und 50 % der CD - Bildungsaktivität des Ausgangsenzyms (Biochemistry. (1994) 33 (33), S. 9929-9936, Biochemistry. (1995) 34 (10), S. 3368-3376)

Aufgabe der Erfindung war es, eine Cyclodextringlycosyltransferase-Mutante (CGTase-Mutante) zur Verfügung zu stellen, welche bei der Umsetzung von Stärke oder stärkeähnlichen Substraten zu CD in höherem Ausmaß als die Ausgangs-CGTase $\gamma$-CD produziert und die noch mindestens 60 der spezifischen Gesamt-CGTase-Aktivität der Ausgangs-CGTase aufweist.

Eine weitere Aufgabe der Erfindung war es, Verfahren zur Herstellung der genannten CGTase-Mutanten zur Verfügung zu stellen.

Eine weitere Aufgabe der Erfindung war es, Verfahren zur Produktion von $\gamma$-CD zur Verfügung zu stellen.

Die erstgenannte Aufgabe wird gelöst durch CGTase-Mutanten, die dadurch gekennzeichnet sind, daß sich ihre Aminosäuresequenz von der Aminosäuresequenz der Ausgangs-CGTase im Bereich von Aminosäureposition 155 bis einschließlich Aminosäureposition 195 durch die Deletion mindestens einer Aminosäure unterscheidet, wobei Position 1 der Proteinsequenz der Beginn des Signalpeptids der Ausgangs-CGTase ist und die Deletion eine höhere γ-CGTase-Aktivität bewirkt.

Im Sinne der Erfindung ist unter einer höheren γ-CGTase-Aktivität zu verstehen, daß im Produktgemisch, welches bei der Umsetzung von Stärke oder stärkeähnlichen Substraten mit CGTasen entsteht, der Quotient

$$\frac{\text{Menge gebildetes } \gamma\text{-CD}}{(\text{Menge gebildetes } \alpha\text{-CD} + \text{Menge gebildetes } \beta\text{-CD})}$$

größer wird.

Bevorzugt unterscheidet sich die Aminosäuresequenz einer erfindungsgemäßen CGTase-Mutante von der Aminosäuresequenz der Ausgangs-CGTase dadurch, daß im Bereich zwischen Aminosäureposition 155 und Aminosäureposition 195 der Ausgangs-CGTase zwischen vier und acht Aminosäurereste deletiert sind, wobei Position 1 der Proteinsequenz der Beginn des Signalpeptids der Ausgangs-CGTase ist und die Deletion eine höhere γ-CGTase-Aktivität der Mutante gegenüber der Ausgangs-CGTase bewirkt.

Besonders bevorzugt unterscheidet sich die Aminosäuresequenz einer erfindungsgemäßen CGTase-Mutante von der Aminosäuresequenz der Ausgangs-CGTase dadurch, daß im Bereich zwischen Aminosäureposition 155 und Aminosäureposition 195 der Ausgangs-CGTase sechs Aminosäurereste deletiert sind, wobei Position 1 der Proteinsequenz der Beginn des Signalpeptids der Ausgangs-CGTase ist und die Deletion eine höhere γ-CGTase-Aktivität der Mutante gegenüber der Ausgangs-CGTase bewirkt.

Auch für die weiteren in der Anmeldung genannten Aminosäurepositionen gilt jeweils, daß Position 1 der Proteinsequenz der Beginn des Signalpeptids der Ausgangs-CGTase ist.

Insbesondere bevorzugt ist ferner eine CGTase-Mutante, die dadurch gekennzeichnet ist, daß die in Tab. 1 genannten CGTasen die Ausgangs-CGTasen sind und daß sich ihre Aminosäuresequenz von der jeweiligen Aminosäuresequenz der in Tab. 1 genannten Ausgangs-CGTase zumindest durch die Deletion der jeweils fettgedruckten Aminosäurereste unterscheidet, wobei die übrige Aminosäuresequenz der Mutante zur jeweiligen Aminosäuresequenz der Ausgangs-CGTase soweit homolog ist, daß die übrige Sequenz CGTase-Aktivität aufweist.

Beispiele für erfindungsgemäße CGTase-Mutanten sind CGTasen, die aus den in Tab. 1 aufgeführten CGTasen oder aus anderen CGTasen durch Deletion einzelner Aminosäurereste im Bereich zwischen den Aminosäureresten 155 und 195 erhalten werden. Bevorzugt sind CGTasen, bei denen vier bis acht Reste im genannten Bereich deletiert sind. Besonders bevorzugt sind CGTasen, bei denen die in Tabelle 1 durch Fettdruck markierten sechs Aminosäurereste im genannten Bereich deletiert sind.

Weitere Beispiele für erfindungsgemäße CGTase-Mutanten sind Enzyme, bei welchen die zu den in Tab. 1 genannten Aminosäuren homologen Aminosäuren deletiert sind, wobei diese Enzyme ohne die erfindungsgemäße Deletion CGTase Aktivität aufweisen.

Beispiele für erfindungsgemäße CGTase-Mutanten sind ferner Enzyme, bei welchen die in Tabelle 1 jeweils fettgedruckten Aminosäurereste im Bereich zwischen Aminosäureposition 155 und Aminosäureposition 195 deletiert sind, wobei die übrige Aminosäuresequenz der erfindungsgemäßen CGTase-Mutante zur jeweiligen Aminosäuresequenz der Ausgangs-CGTase aus dem jeweils in Tabelle 1 genannten Mikroorganismus soweit homolog ist, daß das Ausgangs-Enzym CGTase-Aktivität aufweist.

**Tabelle 1:**

| ß-CGTase aus | Position | Aminosäuresequenz | | | |
|---|---|---|---|---|---|
| Bacillus ohbensis | 160 | -PNHSSPA | LETDPS | YAENGAVYNDG- | (Seq. ID. No 1) |
| Bacillus macerans | 165 | -PNHTSPA | DRDNPG | FAENGGMYDNG- | (Seq. ID. No 2) |
| Bacillus sp. 1-1 | 160 | -PNHSSPA | LETNPN | YVENGAIYDNG- | (Seq. ID. No 3) |
| Bacillus circulans #8 | 172 | -PNHTSPA | METDTS | FAENGRLYDNG- | (Seq. ID. No 4) |

EP 0 802 259 B1

Die erfindungsgemäßen CGTase-Mutanten besitzen unerwarteterweise eine höhere γ-CD Spezifität als die zu ihrer Herstellung verwendeten Ausgangs-CGTasen bei einer gleichzeitig nicht wesentlichen Reduktion der spezifischen Gesamt-CGTase-Aktivität des mutierten Enzyms im Vergleich zur Ausgang-CGTase.

Die erfindungsgemäßen CGTase-Mutanten produzieren somit bei der Umsetzung von Stärke oder stärkeähnlichen Substraten CD's in einer Produktverteilung, bei der der Quotient aus γ-CD und der Summe aus α-CD und β-CD größer ist als der Quotient dieser Produkte, der bei der Stärkeumsetzung mit der jeweiligen unveränderten Ausgangs-CGTase erzielt wird.

Die Aufstellung in Tab.1 zeigt beispielhaft anhand einiger Ausgangs-CGTasen den in CGTasen generell vorhandenen, homologen Aminosäuresequenzbereich, sowie für die Modifikation der Produktspezifität jeweils relevante sechs Aminosäurereste innerhalb dieses Sequenzbereiches.

Als Position ist in Tab. 1 die Zahl der ersten Aminosäure der jeweils wiedergegebenen Aminosäuresequenz bezeichnet, wobei als Position 1 die erste Aminosäure des Signalpeptids der jeweiligen Ausgangs-CGTase Sequenz gezählt wurde. Anhand von allgemein bekannten Standardverfahren läßt sich der entsprechende Sequenzbereich bei allen CGTasen eruieren. Dies ist beispielsweise mittels bekannter Algorhithmen, die multiple Sequenzalignments berechnen, möglich. Ein Beispiel für einen geeigneten Computeralgorhithmus ist das Programm "pileup" aus dem kommerziell erhältlichen Sequenzanalyseprogramm Wisconsin Sequence Analysis Package (Genetic Computer Group, Madison).

Durch Mutagenese des dargestellten Bereiches in Ausgangs-CGTasen lassen sich mittels bekannter Standardverfahren, wie sie beispielhaft auch in der vorliegenden Anmeldung ausgeführt sind, erfindungsgemäße Enzyme aus beliebigen CGTasen herstellen. Dazu wird in der Regel ein für eine Ausgangs-CGTase kodierendes Gen derart mutiert, daß es für eine erfindungsgemäße CGTase-Mutante kodiert.

Die Erfindung betrifft somit auch Verfahren zur Herstellung von mutierten CGTase-Genen, welche für CGTase-Mutanten der Ansprüche 1 - 3 kodieren, dadurch gekennzeichnet, daß die DNS-Sequenz eines für eine Ausgangs-CGTase kodierenden Gens mittels an sich bekannter Mutagenesemethoden derart mutiert wird, daß sich die durch die DNS-Sequenz des mutierten Gens kodierte Aminosäuresequenz im Bereich zwischen Aminosäureposition 155 und 195 von der durch die DNS das unmutierten Gens kodierten Aminosäuresequenz durch die Deletion mindestens eines Aminosäurerestes unterscheidet.

Vorzugsweise wird im erfindungsgemäßen Verfahren die DNS-Sequenz eines für eine Ausgangs-CGTase kodierenden Gens mittels an sich bekannter Mutagenesemethoden derart mutiert, daß die durch die DNS-Sequenz des mutierten Gens kodierte Aminosäuresequenz sich von der durch die DNS des unmutierten Gens kodierten Aminosäuresequenz durch die Deletion von vier bis acht Aminosäureresten im Bereich zwischen Aminosäureposition 155 und 195 unterscheidet.

Besonders bevorzugt wird im erfindungsgemäßen Verfahren die DNS-Sequenz eines für eine Ausgangs-CGTase kodierenden Gens mittels an sich bekannter Mutagenesemethoden derart mutiert, daß die durch die DNS-Sequenz des mutierten Gens kodierte Aminosäuresequenz sich von der durch die DNS des unmutierten Gens kodierten Aminosäuresequenz durch die Deletion von sechs Aminosäureresten im Bereich zwischen Aminosäureposition 155 und 195 unterscheidet.

Die Erfindung betrifft ferner Verfahren zur Herstellung von γ-CGTasen dadurch gekennzeichnet, daß mindestens eine der beschriebenen DNS-Sequenzen in einem Mikroorganismus exprimiert wird.

Zur Herstellung der erfindungsgemäßen CGTasen sind die Gene aller CGTasen (Ausgangs-CGTasen) geeignet. Ausgangs-CGTasen können alle natürlicherweise vorkommenden CGTasen sein, aber auch durch Mutagenese erhaltene CGTasen wie zum Beispiel solche CGTasen, bei denen das Produktbildungsverhältnis durch eine nicht erfindungsgemäße, andere Mutation (z. B.: wie in DE 43 24 650 A1, entspricht US-A-5,474,917, beschrieben) bereits verändert wurde. Ausgangs-CGTasen sind bevorzugt solche CGTasen, bei denen das Produktbildungsverhältnis durch eine nicht erfindungsgemäße, andere Mutation (z. B.: wie in DE 43 24 650 Al beschrieben) bereits verändert wurde.

Das für eine Ausgangs-CGTase kodierende Gen wird mittels bekannter Verfahren isoliert und die erfindungsgemäße Mutation wird in das Gen der CGTase durch "in vivo"- oder "in vitro"-Mutageneseverfahren eingeführt. Solche Verfahren sind ebenfalls im Stand der Technik allgemein bekannt.

Unter "in vivo"-Mutageneseverfahren sind besonders solche Methoden zu verstehen, bei denen Mikroorganismen, die chromosomal und/oder episomal ein für eine CGTase kodierendes Gen enthalten, mit einem Mutagen wie z. B. UV-Licht, Nitrosoguanidin oder Ethylmethylsulfonat unspezifisch mutagenisiert werden. Ein solches Verfahren ist beispielsweise von Miller J. H. in (1972) Experiments in Molecular Genetics; Cold Spring Harbor Laboratory; Cold Spring Harbor, N.Y. beschrieben worden.

Anschließend werden durch bekannte Methoden wie beispielsweise der Sequenzanalyse nach der von Sanger et al. in PNAS 74 (1977) 5463-5467 beschriebenen Kettenabbruchmethode Mutanten identifiziert, bei denen im Bereich zwischen den Aminosäureresten 155 und 195 der entsprechenden CGTase zumindest ein für einen Aminosäurerest kodierendes Codon des CGTase-Gens deletiert ist.

Erfindungsgemäß bevorzugt werden unter den Mutanten solche ausgewählt, bei denen im genannten Bereich vier bis acht Codonen deletiert sind.

Besonders bevorzugt werden Mutanten ausgewählt, bei denen sechs Codonen deletiert sind, wobei nochmals bevorzugt solche Mutanten ausgewählt werden, bei denen die sechs Codonen deletiert sind, die für die in Tabelle 1 fettgedruckten Aminosäurereste oder die zu diesen homologen Aminosäurereste in anderen CGTasen kodieren.

Unter "in vitro"-Mutagenesemethoden sind im Sinne der Erfindung solche Methoden zu verstehen, bei denen ein isoliertes CGTase-Gen oder ein Fragment eines CGTase-Gens in an sich bekannter Art und Weise so modifiziert wird, daß ein Gen entsteht, welches für ein CGTase-Enzym kodiert, bei dem im Bereich zwischen den Aminosäureresten 155 und 195 zumindest ein für einen dieser Aminosäurereste kodierendes Codon des CGTase-Gens deletiert ist.

Bevorzugt sind Mutanten, die so modifiziert wurden, daß im genannten Bereich vier bis acht Codonen deletiert sind. Besonders bevorzugt sind Mutanten, die so modifiziert wurden, daß im genannten Bereich sechs Codonen deletiert sind, wobei insbesondere Mutanten, die so modifiziert wurden, daß im genannten Bereich die sechs Codonen deletiert sind, die für die in Tabelle 1 fettgedruckten oder die zu diesen homologen Aminosäurereste in anderen CGTasen kodieren, besonders bevorzugt sind.

Die Erfindung betrifft somit auch DNS-Sequenzen, die für erfindungsgemäße γ-CGTase-Mutanten kodieren.

Aus dem Stand der Technik bekannte Verfahren zur "in vitro"-Mutagenese sind z. B. spezifische (BioTechniques (1992)13 (3), S. 342-346) oder unspezifische (Technique (1989) 1 (1), S. 11-15) Mutageneseverfahren mit Hilfe der "PCR"-Technik. Ebenso sind Verfahren bekannt, bei denen die Mutation gerichtet mit Hilfe eines synthetisierten Oligonukleotids in das Zielgen eingebracht wird. Dies kann sowohl mittels sogenannter "Einzelstrangverfahren" (Ausubel F.M et al. (1987) Current Protocols in Molecular Biology, Green Publishing Associates) oder auch mittels "Doppelstrangverfahren" (Promega 1992-1993 Catalogue, 150) oder mittels anderer Verfahren, wie sie beispielsweise in Ann. Rev. Genet. (1985) 19, S. 423-462 beschrieben werden, geschehen.

Das Hauptanwendungsgebiet der erfindungsgemäßen CGTase-Mutante ist ihre Verwendung zur Gewinnung von γ-CD aus Stärke. Die erfindungsgemäße CGTase-Mutante lässt sich dazu mittels gängiger Herstellungsverfahren nutzen.

Die Erfindung betrifft somit auch Verfahren zur Herstellung von γ-CD durch Umsetzung von Stärke mittels einer CGTase, dadurch gekennzeichnet, daß als CGTase mindestens eine CGTase-Mutante gemäß einem der Ansprüche 1 bis 4 eingesetzt wird.

Gängige Herstellungsverfahren zur Produktion von γ-CD, in denen sich die erfindungsgemäßen CGTasen anstelle der dort genannten CGTasen einsetzen lassen, sind zum Beispiel beschrieben bei:

- Journal of Fermentation and Bioengineering (1990) 70 (3), S. 190-192: Die Herstellung von γ-CD unter Verwendung der β-, γ-CD bildenden CGTase aus Bacillus sp. AL-6 in Gegenwart von Äthanol, welcher eine verstärkte γ-CD-Produktion bewirkt.

- CA 107:57466 beschreibt die Herstellung von γ-CD unter Verwendung der γ-CGTase aus Bacillus sp. 313.

- EP 291,067: Herstellung von γ-CD unter Verwendung der CGTase aus Bacillus macerans. Die Produktspezifität für γ-CD wird durch die Zugabe eines Komplexbildners, z.B. Cyclohexadec-8-en-1-on, erreicht.

- DE 40 09 822 (entspricht US-A-5,409,824): Produktion von γ-CD mit der γ-CGTase aus Bacillus sp. 290-3.

γ-CD besitzt sowohl im Vergleich zu α-CD als auch im Vergleich zu β-CD spezifische Vorteile, die es für eine Reihe von Anwendungen als einzig mögliches oder am besten geeignetes CD ausweisen.

Im Vergleich zu α-CD, das aus sechs Glukoseeinheiten aufgebaut ist, besitzt das aus acht Glukoseeinheiten bestehende γ-CD eine größere hydrophobe Kavität, die auch eine Komplexierung solcher Gastmoleküle ermöglicht, die aus sterischen Gründen von α-CD nicht komplexiert werden können.

Im Vergleich zu β-CD (Löslichkeit in Wasser bei Raumtemperatur: ca. 18,5 g/l) besitzt γ-CD eine wesentlich höhere Löslichkeit (bei Raumtemperatur: ca. 232,0 g/l) und ist damit für Komplexierungsreaktionen aus wäßrigen Lösungen besser geeignet als β-CD. Ein weiterer Vorteil von γ-CD gegenüber β-CD und modifizierten β-CD-Derivaten ist die geringe Toxizität von γ-CD. Sowohl bei oraler als auch bei intravenöser Applikation sind α-CD- und β-CD-Derivate im Tiermodell toxischer als γ-CD.

Die folgenden Beispiele dienen der weiteren Erläuterung der Erfindung.

**Beispiel 1: Mutagenese der CGTase aus Bacillus circulans #8 (DSM 10559)**

Die Deletion beliebiger Aminosäurereste im erfindungsgemäßen Bereich der Aminosäurereste 155 - 195, insbesondere die Deletion der sechs Aminosäurereste M E T D T S (Seq. ID No. 5) an der Position 179-184 in der β-CGTase aus Bacillus circulans #8 (vgl. Tab. 1; Hinterlegt bei der DSMZ Deutschen Sammlung von Mikroorganismen und Zellkulturen in Braunschweig unter der Nummer **DSM 10559**) wird erreicht, indem die für die entsprechenden Aminosäurereste kodierenden Basentriplets des CGTase-Strukturgens in einer dem Fachmann an sich bekannten Art und Weise deletiert werden.

Zur Mutagenese wurde das β-CGTase-Gen aus Bacillus circulans #8 zunächst in den kommerziell erhältlichen E. coli-Vektor pUC19 (Boehringer, Mannheim) kloniert. Dazu wurde wie bei Ausubel F.M., Current protocols in molecular biology, vol. 1; Greene Publishing Associates & Wiley - Interscience, N. Y. beschrieben chromosomale DNS aus Bacillus circulans #8 (Appl. Microbiol. Biotechnol. (1990) 33: Seite 542 - 546) isoliert und mit den Restriktionsendonukleasen HindIII und Xbal (Boehringer, Mannheim) gespalten. Fragmente in einem Größenbereich zwischen zwei und fünf kb wurden isoliert und zusammen mit einer mit den Restriktionsendonukleasen HindIII und Xbal (Boehringer, Mannheim) gespaltenen pUC19-DNS und T4 DNS-Ligase bei 16°C für 12 Stunden inkubiert. Der Ligationsansatz wurde zur Transformation von E. coli K 12-Zellen , die mit bekannten Verfahren (Maniatis, Molecular Cloning, A Laboratory Manual; Cold Spring Harbor Laboratory (1982), N.Y.) kompetent für die Aufnahme von DNS gemacht wurden, verwendet. Aus solchen E. coli-Zellen, die nach der Transformation auf Stärke enthaltenden Indikatorplatten Stärkeabbauhöfe bildeten, wurde das rekombinante Plasmid, welches das Gen für die β-CGTase aus Bacillus circulans #8 trägt, isoliert (Maniatis, Molecular Cloning, A Laboratory Manual; Cold Spring Harbor Laboratory (1982), N.Y., S. 86-92).

Die Mutagenese dieses Gens wurde mit dem von der Firma Amersham (Braunschweig) kommerziell vertriebenen 'Oligonucleotide - directed in vitro mutagenesis system, version 2.1', basierend auf einem von Eckstein entwikkelten Verfahren (Nucl. Acids. Res. (1986) 14, S. 9679-9698 und Nucl. Acids. Res. (1988)16, S. 791-802) durchgeführt. Die Mutagenese wurde exakt nach dem Protokoll durchgeführt, welches dem genannten Mutagenesesystem der Fa. Amersham beiliegt. Das Verfahren wird im folgenden summarisch wiedergegeben. Details sind dem Protokoll des genannten Mutagenesesystems zu entnehmen.

Unter Verwendung kommerziell erhältlicher Enzyme wie Restriktionsendonukleasen und T4 DNS-Ligase (Boehringer, Mannheim) wurde ein solcher Teil des in pUC19 klonierten Gens für die β-CGTase aus Bacillus circulans #8, der für den erfindungsgemäßen Bereich von Aminosäurerest 155 bis 195 dieser CGTase kodiert, in den kommerziell erhältlichen Vektor M13 (New England Biolabs) kloniert. Ein Beispiel für ein solches Fragment ist ein 1,6 kb großes Accl-Fragment. Dieses Fragment wurde in den mit der Restriktionsendonuklease Accl gespaltenen M13-Vektor kloniert.

Aus solchen E. coli-Wirtszellen, die den rekombinanten M13-Vektor aufgenommen hatten, wurde nach der von Amersham mit dem oben genannten Mutagenese-System gelieferten Versuchsprotokoll einzelsträngige, rekombinante M13-DNS (Vorlagen-DNS) isoliert.

Zur eigentlichen Mutagenese wurden chemisch definierte Mutageneseoligonukleotide mit jeweils erwünschter Sequenz synthetisiert. Solche Oligonukleotide sind beispielsweise bei der Fa. MWG (Ebersberg) käuflich erhältlich. Die Sequenz des Mutageneseoligonukleotids wurde so gewählt, daß die Abfolge der Basen in dem Mutageneseoligonukleotid invers komplementär zu dem Teil der Nukleotidsequenz der Vorlagen-DNS ist, der die in der Vorlagen-DNS enthaltenen, zu deletierenden Basentriplets der β-CGTase aus Bacillus circulans #8 jeweils 15 Basen stromauf und stromab umgibt.

In Tab. 2 ist die Sequenzen des verwendeten Mutageneseoligonukleotids dargestellt.

**Tab.2:**

**5'- CAC ACC TCT CCA GCG TTT GCC GAA AAT GGC -3' (Seq. ID.No 6)**

Der Einsatz des in Tab.2 dargestellten Mutageneseoligonukleotids führte zu einem β-CGTase-Genfragment, das für ein Aminosäurefragment kodiert, bei dem die sechs Aminosäurereste M E T D T S (Seq. ID No. 5) deletiert sind.

Das Mutageneseoligonukleotid wurde am 5'-Ende unter Verwendung von T4 Polynukleotid-Kinase und ATP (Amersham) phosphoryliert. Das phosphorylierte Mutageneseoligonukleotid wurde an die homologen Bereiche der Vorlagen-DNS gebunden. Dazu wurden 5 μg einzelsträngiger Vorlagen-DNS mit ca. 4 pMol des phosphorylierten Mutageneseoligonukleotids für drei Minuten bei 70°C und dann für 30 Minuten bei 37°C inkubiert. Anschließend erfolgte die Synthese eines zur Vorlagen-DNS, mit Ausnahme der zu deletierenden Nukleotide, komplementären DNS-Strangs, wobei das an die Vorlagen-DNS gebundene Mutageneseoligonukleotid als Startpunkt der Synthese und die Vorlagen-DNS als Vorlage für die Neusynthese des mutierten DNS-Strangs diente. Die Synthese selbst erfolgte nach Zugabe des Klenow-Fragments der DNS-Polymerase (Amersham), einer T4 DNS-Ligase und eines Nukleotidmixes, der die Nukleotide dATP, dGTP, dTTP und anstelle des dCTP das Thionukleotid dCTPS (Amersham) enthält, über 15 Stunden bei 16°C.

Aus diesem Syntheseansatz wurden verbliebene Moleküle einzelsträngiger Vorlagen-DNS entfernt. Dazu wurde der Ansatz mit NaCl versetzt und über einen Nitrozellulosefilter (Amersham) filtriert, der spezifisch einzelsträngige DNS bindet. Die im Durchlauf verbliebene doppelsträngige Hybrid-DNS wurde durch eine EtOH-Fällung konzentriert und entsalzt. Anschließend wurde die Hybrid-DNS mit NciI (Amersham), einer Restriktionsendonuklease, die die Nukleotidsequenz CC(G/C)GG erkennt, aber nur native DNS-Stränge, nicht jedoch solche, die das Nukleotid-Analogon dCTPS enthalten, spaltet, in geeignetem Inkubationspuffer (Amersham) für 90 Minuten bei 37°C inkubiert. Durch diese Behandlung wurden nur in den nicht mutagenisierten Strang (Vorlagen-DNS) Brüche eingeführt.

Bei einer 30 minütigen Behandlung bei 37°C mit Exonuklease III (Amersham), einem Enzym, das DNS-Stränge von freien Enden her abbaut, wurde die Vorlagen-DNS dann entfernt. Nach einer thermischen Inaktivierung der Exonuklease III (15 Minuten bei 70°C) wurde der verbliebene, einzelsträngige und mutagenisierte DNS-Strang mit DNS-Polymerase I (Amersham), T4 DNS-Ligase und den Nukleotiden dATP, dTTP, dCTP und dGTP für 3 Stunden bei 16°C inkubiert. Dabei wurde die mutagenisierte Einzelstrang-DNS zum Doppelstrang ergänzt. Nach einer weiteren EtOH-Fällung zur Reinigung wurde die mutagenisierte DNS in kompetente E. coli K12-Zellen transformiert.

Durch die Sequenz analyse des betreffenden Bereichs der rekombinanten DNS aus fünf der bei der Transformation erhaltenen Klone wurde der Erfolg der Mutageneseprozedur kontrolliert. Aus einem Vektor, bei dem eine Mutation bestätigt wurde, wurde das zur Mutagenese ursprünglich in M13 klonierte DNS-Fragment mit den Restriktionsenzymen XhoI und NdeI herausgespalten.

Anschließend wurde das entsprechende, jedoch nicht mutagenisierte XhoI/NdeI-Fragment aus dem auf pUC19 basierenden Expressionsplasmid für die β-CGTase aus Bacillus circulans #8 herausgespalten und unter Verwendung von T4 DNS-Ligase durch das mutagenisierte Fragment ersetzt.

## Beispiel 2: Mutagenese der β-CGTase aus Bacillus sp. 1-1

Analog zu der in Beispiel 1 dargestellten Methode wurden die sechs Codonen des β-CGTase-Gens aus Bacillus sp. 1-1, die für die Aminosäurereste L E T N P N (Seq. ID No. 7) an Position 167 - 172 der entsprechenden CGTase (vgl. Tab. 1) kodieren, unter Verwendung des in Tabelle 3 dargestellten Oligonukleotides deletiert (A) in Bsp. 8, Vergleich 2). Die gleiche Deletion wurde auch in ein in DE 43 24 650 AI beschriebenes Derivat dieser CGTase, bei dem durch einen Aminosäureaustausch (Tyr => Trp) die γ-CD - Spezifität im Vergleich zum Wildtypenzym bereits erhöht wurde, eingebracht (B) in Bsp.8, Vergleich 2)

```
Tab. 3:


5'- CAT TCA TCA CCG GCA TAT GTT GAA AAT GGG -3'  (Seq.ID No. 8)
```

## Beispiel 3: Produktion der β-CGTase aus Bacillus circulans #8 und ihrer erfindungsgemäßen Derivate in E. coli

Zur Produktion der β-CGTase aus Bacillus circulans #8 und ihres gemäß Bsp. 1 hergestellten Derivats wurden die in Beispiel 1 beschriebenen Expressionsplasmide auf pUC19-Basis in einen E. coli-Sekretionsstamm transformiert. Als E. coli-Sekretionsstamm wurde E. coli WCM105 verwendet. Dieser Stamm wurde, wie in EP 338410 beschrieben, aus E. coli DS 410 hergestellt.

Zur Produktion der β-CGTase aus Bacillus circulans #8 oder deren Derivat wurden daher Zellen von E. coli WCM105, die geeignete CGTase-Expressionsplasmide enthalten, in LB-Medium (Maniatis, Molecular Cloning, A Laboratory Manual; Cold Spring Harbor Laboratory (1982), N.Y.), das 10 g/l Laktose und 0,1 g/l Ampicillin enthält, für 72 Stunden bei 30°C in einem Wasserbadschüttler (Drehzahl 250 Upm) inkubiert. Dann wurden die Zellen durch Zentrifugation bei 5000 x g abgetrennt. Der zellfreie Kulturüberstand enthält die β-CGTase oder deren Derivate.

## Beispiel 4: Produktion der β-CGTase aus Bacillus sp. 1-1 und ihrer erfindungsgemäßen Derivate in E. coli

Die Produktion erfolgte analog Beispiel 3 unter Verwendung oder in Bsp. 2 beschriebenen Expressionsplasmide.

## Beispiel 5: Reinigung von CGTasen mittels Adsorption an trägergebundenes ß-Cyclodextrin

Eine spezifische und schonende Reinigung von CGTasen erfolgt über eine Affinitätsreinigung mittels an Sepharose gekoppelte β-CD - Moleküle.

1 g Epoxy - aktivierte Sepharose 6B (Sigma) wird mit 3 x je 10 ml $H_2O$ und anschließend 1 x 5 ml 0,1 N NaOH

EP 0 802 259 B1

gewaschen. Anschließend wird die Sepharose 6B in 2 ml einer 2,8 % igen (w/v) Lösung aus β-CD in 0,1 NaOH suspendiert und 20 h bei 45°C unter leichtem Schütteln inkubiert. Dann wird das Kopplungsprodukt aus β-CD und Sephadex 6B mit 2 x 5 ml $H_2O$ gewaschen. Nach Suspendierung des gewaschenen Materials in einer 2,5 % - igen (w/v) Glukoselösung in $H_2O$ wird das Material zur Absättigung der verbliebenen freien Kopplungsstellen für 1 h bei RT inkubiert. Anschließend wird successive mit 2 x 5 ml $H_2O$, 2 x 5 ml 0,1 M Boratpuffer pH 8,0, 2 x 5 ml 0,1 M Acetatpuffer pH 4,0 und 2 x 2 ml 20 mM Triethanolamin/Cl pH 7,2 gewaschen. Das Kopplungsprodukt wird mit 0,2 ml 20 mM Triethanolamin/Cl pH 7,2 versetzt (Endvolumen ca. 2 - 2,5 ml) und bei 4°C bis zum Gebrauch aufbewahrt.

Zur spezifischen Bindung von CGTasen an das an Sepharose 6B gekoppelte β-CD (CD-Sepharose) werden die nach Beispiel 3 oder 4 erhaltenen, zellfreien, CGTase-haltigen Kulturüberstände mit 0,2 ml der CD-Sepharose versetzt und für 1,5 h bei 4°C unter leichtem Schütteln inkubiert. Dabei koppelt das Enzym an die CD-Sepharose. Der Enzym - CD-Sepharose - Komplex wird durch Zentrifugation (5 min 4000 x g) gewonnen und mit 2 x 10 ml 20 mM Triethanolamin/Cl pH 7,2 gewaschen. Anschließend wird das CGTase - Enzym durch Inkubation des Komplexes für 1,5 h bei 4°C mit 2 ml einer 1%-igen β-CD - Lösung in 20 mM Triethanolamin/Cl pH 7,2 eluiert. Nach einer abschließenden Zentrifugation (5 min, 4000 x g) wird der die gereinigte CGTase enthaltende Überstand abgenommen.

Vor einer Charakterisierung der so gereinigten CGTasen muß noch das in der Lösung enthaltene, zur Elution verwendete β-CD entfernt werden. Dazu wird eine kommerziell erhältliche PD-10 - Säule (Sephadex G-25 M; Pharmacia) mit 35 ml 20 mM Tris/Cl pH 7,2 und 5 mM $CaCl_2$ (TC-Puffer) equilibriert. Die β-CD - haltige Lösung wird mit TC-Puffer auf ein Volumen von 2,5 ml aufgefüllt und auf die Säule aufgetragen. Anschließend wird mit 3,5 ml TC-Puffer eluiert. Das dabei erhaltene Eluat enthält die gereinigte, β-CD - freie CGTase.

## Beispiel 6: Konversion von Stärke zu Cyclodextrinen

Die Bestimmung der Aktivitäten von CGTasen erfolgte nach der in Eur. J. Biochem. (1990) 191, S. 177-185 beschriebenen Methode.

Verschiedene Mengen einer zu testenden CGTase-Lösung wurden mit einer 5 %-igen Lösung einer löslichen Stärke (Merck, Darmstadt) in einem Puffer bestehend aus 20 mM Tris/HCl pH 7,2 und 5 mM $CaCl_2$ für eine definierte Zeit bei 45°C inkubiert. Nach der Zeit wurde die Reaktion durch die Zugabe von 1,5 Volumenteilen Methanol beendet. Nicht umgesetzte Reststärke wurde durch eine einstündige Inkubation bei 4°C gefällt und durch Zentrifugation (10 min, 12000 x g) abgetrennt. Die entstandenen Produkte wurden über HPLC an einer Nukleosil 10-$NH_2$-Säule (Macherey & Nagel, Düren) bestimmt, wobei definierte Cyclodextrine (Sigma, München) als Standard dienten. Als eine Einheit (1 U) wird die Enzymmenge definiert, die unter den beschriebenen Bedingungen 1 μM Cyclodextrine pro Minute aus Stärke bildet.

## Beispiel 7: Die Bestimmung der spezifischen Gesamt-CGTase-Aktivitäten von gereinigten CGTasen

Die spezifische Gesamt-CGTase-Aktivität gereinigter CGTasen ist definiert als Volumenaktivität pro Proteinmenge (U/mg).

Die CGTase - Volumenaktivität (U/ml) einer Enzymprobe wird, wie in Beispiel 6 beschrieben, ermittelt.

Der Proteingehalt (mg/ml) einer Lösung der gereinigten CGTase wird nach der von M. Bradford (Anal. Biochem. (1976) 72, Seite 248 ff) beschriebenen Methode durchgeführt. Die dazu benötigten Chemikalien werden von der Firma Bio-Rad bezogen.

Für die β-CGTasen aus Bacillus circulans #8 und Bacillus sp. 1-1, sowie die daraus (wie in Beispiel 1 und Beispiel 2 beschrieben) erzeugten Deletionsmutanten wurden folgende spezifische Gesamt-CGTase-Aktivitäten (spez. Aktivität) ermittelt:

| Enzym | rel. Aktivität (%) | spez. Aktivität (U/mg) |
|---|---|---|
| Wildtyp CGTase aus Bacillus circulans #8 | 100 | 106 |
| davon abgeleitete Deletionsmutante | 77 | 82 |
| Wildtyp CGTase aus Bacillus sp. 1-1 | 100 | 120 |
| davon abgeleitete Deletionsmutante | 65 | 78 |
| Mutiertes Derivat (TyrTrp) der CGTase aus Bacillus sp.1-1 | 100 | 23 |
| davon abgeleitete Deletionsmutante | 73 | 17 |

**Beispiel 8: Konversion von Stärke unter Einsatz der nicht mutagenisierten β-CGTasen aus Bacillus circulans #8 und Bacillus sp. 1-1, sowie den gemäß Bsp. 3 und 4 hergestellten Derivaten.**

Zum Vergleich der Produkt spektren der nicht mutagenisierten CGTasen mit den jeweils, wie in den Beispielen 1 und 2 beschrieben, durch Deletionsmutagenese daraus erhaltenen Derivate wurden gleiche Enzymaktivitäten zur Stärkekonversion (Beispiel 6) eingesetzt. Zu definierten Zeitpunkten wurde die Produktzusammensetzung wie beschrieben untersucht. Es wurden folgende Resultate erhalten:

| Vergleich 1: Bacillus circulans #8 | | | | | | | |
|---|---|---|---|---|---|---|---|
| Zeit (min) | Wildtyp-CGTase | | | | Deletionsmutante davon | | |
| | $\alpha$-CD (%) | $\beta$-CD (%) | $\gamma$-CD (%) | $\gamma/(\alpha+\beta)$ | $\alpha$-CD (%) | $\beta$-CD (%) | $\gamma$-CD (%) | $\gamma/(\alpha+\beta)$ |
| 5 | 13 | 70 | 17 | 0,2 | 9 | 58,0 | 33 | 0,49 |
| 10 | 13,5 | 70,3 | 16,2 | 0,19 | 8,2 | 60,5 | 31,3 | 0,46 |
| 15 | 11,7 | 66,3 | 22,0 | 0,28 | 9,4 | 62,0 | 28,6 | 0,40 |

| Vergleich 2: Bacillus sp. 1-1 | | | | | | | |
|---|---|---|---|---|---|---|---|
| A) | | | | | | | |
| Zeit (min) | Wildtyp-CGTase | | | | Deletionsmutante davon | | |
| | $\alpha$-CD (%) | $\beta$-CD (%) | $\gamma$-CD (%) | $\gamma/(\alpha+\beta)$ | $\alpha$-CD (%) | $\beta$-CD (%) | $\gamma$-CD (%) | $\gamma/(\alpha+\beta)$ |
| 5 | 0 | 100 | 0 | 0 | 0 | 68 | 32 | 0,47 |
| 10 | 0 | 88 | 12 | 0,14 | 0 | 68 | 32 | 0,47 |
| 15 | 0 | 88 | 12 | 0,14 | 0 | 67,7 | 32,3 | 0,48 |
| B) | | | | | | | |
| Zeit (min) | Wildtyp-CGTase | | | | Deletionsmutante davon | | |
| | $\alpha$-CD (%) | $\beta$-CD (%) | $\gamma$-CD (%) | $\gamma/(\alpha+\beta)$ | $\alpha$-CD (%) | $\beta$-CD (%) | $\gamma$-CD (%) | $\gamma/(\alpha+\beta)$ |
| 5 | 0 | 27 | 73 | 2,7 | 0 | 0 | 100 | - |
| 10 | 0 | 31 | 69 | 2,23 | 0 | 22 | 78 | 3,54 |
| 15 | 0 | 32,5 | 67,5 | 2,08 | 0 | 16,6 | 83,4 | 5,02 |

**Patentansprüche**

1. CGTase-Mutante, welche bei der Umsetzung von Stärke oder stärkeähnlichen Substraten zu CD in höherem Ausmaß als die Ausgangs-CGTase γ-CD produziert und noch mindestens 60 % der spezifischen Gesamt-CGTase-Aktivität der Ausgangs-CGTase aufweist, dadurch gekennzeichnet,daß sich ihre Aminosäuresequenz von der Aminosäuresequenz der Ausgangs-CGTase im Bereich von Aminosäureposition 155 bis einschließlich Aminosäureposition 195 durch die Deletion mindestens einer Aminosäure unterscheidet, wobei Position 1 der Proteinsequenz der Beginn des Signalpeptids der Ausgangs-CGTase ist und die Deletion eine höhere γ-CGTase Aktivität der Mutante gegenüber der Ausgangs-CGTase bewirkt.

2. CGTase-Mutante gemäß Anspruch 1, dadurch gekennzeichnet,daß im Bereich zwischen Aminosäureposition 155 und Aminosäureposition 195 der Ausgangs-CGTase zwischen vier und acht Aminosäurereste deletiert sind, wobei Position 1 der Proteinsequenz der Beginn des Signalpeptids der Ausgangs-CGTase ist und die Deletion eine höhere γ-CGTase Aktivität der Mutante gegenüber der Ausgangs-CGTase bewirkt.

3. CGTase-Mutante gemäß Anspruch 1, dadurch gekennzeichnet, daß im Bereich zwischen Aminosäureposition 155 und Aminosäureposition 195 der Ausgangs-CGTase sechs Aminosäurereste deletiert sind, wobei Position 1 der Proteinsequenz der Beginn des Signalpeptids der Ausgangs-CGTase ist und die Deletion eine höhere γ-CGTase Aktivität der Mutante gegenüber der Ausgangs-CGTase bewirkt.

4. CGTase-Mutante gemäß Anspruch 1, dadurch gekennzeichnet, daß die in Tab. 1 genannten CGTasen die Ausgangs-CGTasen sind, und daß sich ihre Aminosäuresequenz von der jeweiligen den Aminosäuresequenz der in Tab. 1 genannten Ausgangs-CGTase zumindest durch die Deletion der in Tab. 1 jeweils fettgedruckten Aminosäurereste unterscheidet, wobei die übrige Aminosäuresequenz der Mutante zur jeweiligen Aminosäuresequenz der Ausgangs-CGTase soweit homolog ist, daß die übrige Sequenz CGTase Aktivität aufweist.

5. Verfahren zur Herstellung von CGTase Genen, welche für die CGTase-Mutanten der Ansprüche 1 bis 3 kodieren, dadurch gekennzeichnet, daß die DNS-Sequenz eines für eine Ausgangs-CGTase kodierenden Gens mittels an sich bekannter Mutangenesemethoden derart mutiert wird, daß sich die durch die DNS-Sequenz des mutierten Gens kodierte Aminosäuresequenz im Bereich zwischen Aminosäureposition 155 und 195 von der durch die DNS das unmutierten Gens kodierten Aminosäuresequenz durch die Deletion mindestens eines Aminosäurerestes oder von vier bis acht Aminosäureresten, vorzugsweise von sechs Aminosäureresten unterscheidet.

6. DNS-Sequenzen dadurch gekennzeichnet, daß sie für CGTase-Mutanten gemäß Anspruch 1 kodieren.

7. Verfahren zur Herstellung von γ-CGTase-Mutanten dadurch gekennzeichnet, daß mindestens eine DNS-Sequenz gemäß Anspruch 6 in einem Mikroorganismus exprimiert wird.

8. Verfahren zur Herstellung von gamma-CD durch Umsetzung von Stärke mit einer CGTase, dadurch gekennzeichnet, daß als CGTase mindestens eine CGTase-Mutante gemäß einem der Ansprüche 1 bis 4 eingesetzt wird.

**Claims**

1. CGTase mutant which, when converting starch or starch-like substrates into CD, produces γ-CD to a greater extent than the starting CGTase and still exhibits at least 60% of the specific total CGTase activity of the starting CGTase, characterized in that its amino acid sequence differs from the amino acid sequence of the starting CGTase by the deletion of at least one amino acid in the region from amino acid position 155 up to and including amino acid position 195, where position 1 of the protein sequence is the beginning of the signal peptide of the starting CGTase and the deletion brings about increased γ-CGTase activity of the mutant as compared with the starting CGTase.

2. CGTase mutant according to Claim 1, characterized in that between four and eight amino acid residues have been deleted in the region between amino acid position 155 and amino acid position 195 of the starting CGTase, where position 1 of the protein sequence is the beginning of the signal peptide of the starting CGTase and the deletion brings about increased γ-CGTase activity of the mutant as compared with the starting CGTase.

3. CGTase mutant according to Claim 1, characterized in that six amino acid residues have been deleted in the region between amino acid position 155 and amino acid position 195 of the starting CGTase, where position 1 of the protein sequence is the beginning of the signal peptide of the starting CGTase and the deletion brings about increased γ-CGTase activity of the mutant as compared with the starting CGTase.

4. CGTase mutant according to Claim 1, characterized in that the CGTases specified in Tab. 1 are the starting CGTases, and that their amino acid sequence differs from the particular amino acid sequence of the starting CGTase specified in Tab. 1 at least by the deletion of the amino acid residues which are in each case printed in bold, with the remaining amino acid sequence of the mutant being homologous to the particular amino acid sequence of the starting CGTase to the extent that the remaining sequence exhibits CGTase activity.

5. Process for preparing CGTase genes which encode the CGTase mutants of Claims 1 to 3, characterized in that the DNA sequence of a gene encoding a starting CGTase is mutated, by means of mutagenesis methods which are known per se, such that the amino acid sequence encoded by the DNA sequence of the mutated gene differs in the region between amino acid position 155 and 195 from the amino acid sequence encoded by the DNA the [sic] unmutated gene by the deletion of at least one amino acid residue or of four to eight amino acid residues, preferably of six amino acid residues.

6. DNA sequences characterized in that they encode CGTase mutants according to Claim 1.

7. Process for preparing γ-CGTase mutants, characterized in that at least one DNA sequence according to Claim 6 is expressed in a microorganism.

8. Process for preparing gamma-CD by converting starch using a CGTase, characterized in that at least one CGTase mutant according to one of Claims 1 to 4 is employed as the CGTase.

## Revendications

1. Mutant de la CGTase qui, lors de la conversion en CD d'amidon ou de substrats de type amidon, produit la γ-CD dans une mesure supérieure à la CGTase de départ et présente encore au moins 60% de l'activité CGTase spécifique totale de la CGTase de départ, caractérisé en ce que sa séquence d'acide aminé diffère de la séquence d'acide aminé de la CGTase de départ par la délétion d'au moins un acide aminé dans la région allant de la position d'acide aminé 155 jusqu'à et y compris la position d'acide aminé 195, la position 1 de la séquence de protéine étant le début du peptide signal de la CGTase de départ et la délétion provoquant une activité γ-CGTase supérieure du mutant par rapport à la CGTase de départ.

2. Mutant de la CGTase suivant la revendication 1, caractérisé en ce qu'entre quatre et huit résidus d'acide aminé subissent une délétion dans la région comprise entre la position d'acide aminé 155 et la position d'acide aminé 195 de la CGTase de départ, la position 1 de la séquence de protéine étant le début du peptide signal de la CGTase de départ et la délétion provoquant une activité γ-CGTase supérieure du mutant par rapport à la CGTase de départ.

3. Mutant de la CGTase suivant la revendication 1, caractérisé en ce que six résidus d'acide aminé subissent une délétion dans la région comprise entre la position d'acide aminé 155 et la position d'acide aminé 195 de la CGTase de départ, la position 1 de la séquence de protéine étant le début du peptide signal de la CGTase de départ et la délétion provoquant une activité γ-CGTase supérieure du mutant par rapport à la CGTase de départ.

4. Mutant de la CGTase suivant la revendication 1, caractérisé en ce que les CGTases citées dans le tableau I sont les CGTases de départ, et en ce que leurs séquences d'acide aminé diffère au moins par la délétion des résidus d'acide aminé qui sont dans chaque cas imprimés en gras, de la séquence d'acide aminé particulière de la CGTase de départ spécifiée dans le tableau I, la séquence d'acide aminé restante du mutant étant homologue à la séquence d'acide aminé particulière de la CGTase de départ dans la mesure où la séquence restante montre une activité CGTase.

5. Procédé de préparation de gènes de la CGTase qui codent pour les mutants de la CGTase suivant les revendications 1 à 3, caractérisé en ce que la séquence d'ADN d'un gène codant pour une CGTase de départ est mutée par des procédés de mutagenèse qui sont connus en soi, de telle sorte que la séquence d'acide aminé codée par la séquence d'ADN du gène muté diffère de la séquence d'acide aminé codée par l'ADN du gène non muté dans la région située entre les positions d'acide aminé 155 et 195, par la délétion d'au moins un résidu d'acide aminé ou de quatre à huit résidus d'acide aminé, de préférence de six résidus d'acide aminé.

6. Séquences d'ADN, caractérisées en ce qu'elles codent pour des mutants de la CGTase suivant la revendication 1.

7. Procédé de préparation de mutants de la γ-CGTase, caractérisé en ce qu'au moins une séquence d'ADN suivant la revendication 6 est exprimée dans un micro-organisme.

8. Procédé de préparation de gamma-CD par conversion d'amidon en utilisant une CGTase, caractérisé en ce que la CGTase utilisée est au moins un mutant de la CGTase suivant l'une des revendications 1 à 4.

SEQUENZ PROTOKOLL

(1) ALLGEMEINE ANGABEN:

(i) ANMELDER:

(A) NAME: Consortium fuer elektrochemische Industrie GmbH
(B) STRASSE: Zielstattstrasse 20
(C) ORT: Muenchen
(D) BUNDESLAND: Bayern
(E) LAND: Deutschland
(F) POSTLEITZAHL: 81379
(G) TELEFON: 049-89-74844-0
(H) TELEFAX: 049-89-74844-350

(ii) BEZEICHNUNG DER ERFINDUNG: Cyclodextringlykosyltransferasen zur Produktion von gamma-Cyclodextrin

(iii) ANZAHL DER SEQUENZEN: 8

(iv) COMPUTER-LESBARE FASSUNG:

    (A) DATENTRÄGER: Floppy disk
    (B) COMPUTER: IBM PC compatible
    (C) BETRIEBSSYSTEM: PC-DOS/MS-DOS
    (D) SOFTWARE: PatentIn Release #1.0, Version #1.30 (EPA)

(2) ANGABEN ZU SEQ ID NO: 1:

    (i) SEQUENZKENNZEICHEN:

        (A) LÄNGE: 24 Aminosäuren
        (B) ART: Aminosäure
        (C) STRANGFORM:
        (D) TOPOLOGIE: linear

    (ii) ART DES MOLEKÜLS: Peptid

    (iii) HYPOTHETISCH: NEIN

    (v) ART DES FRAGMENTS: inneres Fragment

    (vi) URSPRÜNLICHE HERKUNFT:

        (A) ORGANISMUS: Bacillus ohbensis

    (xi) SEQUENZBESCHREIBUNG: SEQ ID NO: 1:

```
Pro Asn His Ser Ser Pro Ala Leu Glu Thr Asp Pro Ser Tyr Ala Glu
 1               5                  10                  15


Asn Gly Ala Val Tyr Asn Asp Gly
            20
```

(2) ANGABEN ZU SEQ ID NO: 2:

    (i) SEQUENZKENNZEICHEN:

        (A) LÄNGE: 24 Aminosäuren
        (B) ART: Aminosäure
        (C) STRANGFORM:
        (D) TOPOLOGIE: linear

    (ii) ART DES MOLEKÜLS: Peptid

    (iii) HYPOTHETISCH: NEIN

    (v) ART DES FRAGMENTS: inneres Fragment

    (vi) URSPRÜNLICHE HERKUNFT:

        (A) ORGANISMUS: Bacillus macerans

(xi) SEQUENZBESCHREIBUNG: SEQ ID NO: 2:

```
Pro Asn His Thr Ser Pro Ala Asp Arg Asp Asn Pro Gly Phe Ala Glu
1               5                       10                  15

Asn Gly Gly Met Tyr Asp Asn Gly
            20
```

(2) ANGABEN ZU SEQ ID NO: 3:

    (i) SEQUENZKENNZEICHEN:

        (A) LÄNGE: 24 Aminosäuren
        (B) ART: Aminosäure
        (C) STRANGFORM:
        (D) TOPOLOGIE: linear

    (ii) ART DES MOLEKÜLS: Peptid

    (iii) HYPOTHETISCH: NEIN

    (v) ART DES FRAGMENTS: inneres Fragment

    (vi) URSPRÜNLICHE HERKUNFT:

        (A) ORGANISMUS: Bacillus sp. 1-1

    (xi) SEQUENZBESCHREIBUNG: SEQ ID NO: 3:

```
Pro Asn His Ser Ser Pro Ala Leu Glu Thr Asn Pro Asn Tyr Val Glu
1               5                       10                  15

Asn Gly Ala Ile Tyr Asp Asn Gly
            20
```

(2) ANGABEN ZU SEQ ID NO: 4:

    (i) SEQUENZKENNZEICHEN:

        (A) LÄNGE: 24 Aminosäuren
        (B) ART: Aminosäure
        (C) STRANGFORM:
        (D) TOPOLOGIE: linear

    (ii) ART DES MOLEKÜLS: Peptid

    (iii) HYPOTHETISCH: NEIN

    (v) ART DES FRAGMENTS: inneres Fragment

(vi) URSPRÜNLICHE HERKUNFT:

(A) ORGANISMUS: Bacillus circulans #8

(xi) SEQUENZBESCHREIBUNG: SEQ ID NO: 4:


Pro Asn His Thr Ser Pro Ala Met Glu Thr Asp Thr Ser Phe Ala Glu
1               5                   10                  15


Asn Gly Arg Leu Tyr Asp Asn Gly
                    20


(2) ANGABEN ZU SEQ ID NO: 5:

(i) SEQUENZ KENNZEICHEN:

(A) LANGE: 6 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(v) ART DES FRAGMENTS: inneres Fragment

(xi) SEQUENZBESCHREIBUNG: SEQ ID NO: 5:


Met Glu Thr Asp Thr Ser
1               5


(2) ANGABEN ZU SEQ ID NO: 6:

(i) SEQUENZKENNZEICHEN:

(A) LÄNGE: 30 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "synthetisch"

(iii) HYPOTHETISCH: NEIN

(iv) ANTISENSE: NEIN

(viii) POSITION IM GENOM:

(C) EINHEITEN: bp

(xi) SEQUENZBESCHREIBUNG: SEQ ID NO: 6:

CACACCTCTC CAGCGTTTGC CGAAAATGGC

30

(2) ANGABEN ZU SEQ ID NO: 7:

(i) SEQUENZKENNZEICHEN:

(A) LÄNGE: 6 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(v) ART DES FRAGMENTS: inneres Fragment

(xi) SEQUENZBESCHREIBUNG: SEQ ID NO: 7:

Leu Glu Thr Asn Pro Asn

1                    5

(2) ANGABEN ZU SEQ ID NO: 8:

(i) SEQUENZKENNZEICHEN:

(A) LÄNGE: 30 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "synthetisch"

(iii) HYPOTHETISCH: NEIN

(iv) ANTISENSE: NEIN

(viii) POSITION IM GENOM:

(C) EINHEITEN: bp

(xi) SEQUENZBESCHREIBUNG: SEQ ID NO: 8:

CATTCATCAC CGGCATATGT TGAAAATGGG

30